# EUROPEAN PATENT SPECIFICATION

(11) **EP 1 871 468 B1**
(45) Date of publication and mention of the grant of the patent: **28.12.2011**
(21) Application number: 05722198.8
(22) Date of filing: 10.03.2005
(51) Int. Cl.: A61N 1/362, A61N 1/365

(54) **IMPLANTABLE HEART STIMULATOR FOR ENABLING NORMAL ATRIO-VENTRICULAR STIMULATION SEQUENCE IN THE PRESENCE OF AV-NODAL INTERFERENCE**
IMPLANTIERBARER HERZSTIMULATOR ZUR DURCHFÜHRUNG EINER NORMALEN ATRIOVENTRIKULARSTIMULATIONSSEQUENZ BEI VORLIEGEN VON AV-KNOTENSTÖRUNGEN
STIMULATEUR CARDIAQUE IMPLANTABLE PERMETTANT UNE SEQUENCE DE STIMULATION AURICULO-VENTRICULAIRE NORMALE EN PRESENCE D'INTERFERENCES DU NOEUD AV

(43) Date of publication of application: 02.01.2008
(73) Proprietor: St. Jude Medical AB, 175 84 Järfälla (SE)
(72) Inventor: SCHÜLLER, Hans, S-224 67 Lund (SE); LINDGREN, Anders, S-187 45 Täby (SE)
(86) International application number: PCT/SE2005/000351
(87) International publication number: WO 2006/096100

(56) References cited:
- US-A1- 2003 065 365
- US-A1- 2003 097 155
- US-B1- 6 453 192
- US-B1- 6 493 583

## Description

### Field of the invention

The present invention generally relates to an implantable heart stimulator. More particularly, the invention relates to the function of a dual chamber pacemaker system aiming to maintain appropriate atrio-ventricular synchrony also in case of AV-nodal overdrive interference, which otherwise could impede the normal atrial contribution to ventricular filling hereby causing an impairment of a patients hemodynamic conditions.

### Background of the invention

Implantable cardiac stimulation devices (pacemakers) are well established by now with a given place in the treatment of a variety of heart rhythm disorders. The fundamental purpose of pacemaker treatment is to restore and to maintain normal heart rhythm with regard to appropriate rate, atrio-ventricular sequence and conduction delay (the latter two provided the atria are not in the state of chronic fibrillation). The stimulation provided by cardiac pacemakers is based on the interaction with sensed intrinsic or provoked cardiac activity.

In a healthy heart, the sinoatrial node (SA node) serves as a natural pacemaker, which initiates electrical impulses causing depolarisation of the atria followed by a transmission of the signal to the atrioventricular node (AV node) and subsequent depolarisation of the ventricles after an appropriate PR time interval. Maintenance of atrio-ventricular synchrony is of outmost importance for optimal cardiac performance with regard to cardiac output. Loss of AV synchrony reduces cardiac output by 10 to 30 %. It is therefore desirable to provide a dual chamber pacemaker system with appropriate algorithms to maintain or restore normal AV synchrony despite various distortions of the intrinsic cardiac rhythm.

The present invention concentrates on a possible timing disorder that might occur if the AV node begins operating too early after an atrial depolarisation is initiated hereby compromising the heart's natural atrio-ventricular delay. The resulting decreased or absent atrial transport carries the risk for significant lowering of cardiac output. Countermeasures against primary accelerated AV nodal rhythms overriding completely the normal SA node or the atrial stimulation rate are presented in patent US 6,493,583 B1. The present invention, however, refers to any upcoming nodal activity during an ongoing PV/AV interval, which is not uncommon to occur during physical exercise in patients suffering from SA nodal dysfunction. Insufficient response of the SA node to physical activity is known as chronotropic incompetence and can occur as delayed onset of rate increase, insufficient "total" rate increase or a too accelerated rate decrease after a workload is completed. For treatment of SA nodal dysfunction it is common to implant a dual chamber pacemaker system with rate responsive function. In case the artificial increase in atrial stimulation rate and/or the maintenance is not sufficient or suboptimal, the risk prevails intrinsic nodal activity may arise. The occurrence of accelerated nodal activity during an ongoing PV or AV interval will have a negative impact on the ventricular filling time and decrease stroke volume resulting in less optimal hemodynamic conditions. While sudden onset of AV-nodal rhythm with inhibition of a dual chamber system can be counteracted by means of US patent 6,493,583, a slow nodal activity increase with occurrence during normal PV and AV intervals will demand a specific algorithm to increase the actual atrial stimulation rate in order to provide the time required for appropriate atrial transport and thus enabling optimal ventricular filling.
Presently there are several algorithms used for the automatic increase in atrial stimulation rate aiming to suppress atrial tachyarrhythmia. These algorithms are based on the detection of supraventricular extra systoles and would not react on ventricular sensed AV nodal activity.

In US-6,493,583 is disclosed an implantable cardiac stimulation device and method for treating intrinsic ventricular rhythms associated with loss of atrial transport. A rhythm detector is arranged to detect an intrinsic ventricular rhythm lacking in atrial transport when an R-wave detector detects a predetermined number of successive R-waves at a rate below a given rate and when each successive R-wave fails to be preceded by an atrial event, either intrinsic or paced. When this rhythm is detected, a dual chamber pulse generator delivers an atrial pacing pulse to an atrium of the heart prior to each successive R-wave. The atrial pacing pulses are delivered at an AV delay prior to the R-waves and the pacing rate is held constant for a time period. Thereafter, the pacing rate is gradually reduced during a recovery time period until the prevailing base rate is reached or until the end of the recovery time period.

US-6,453,192 relates to a method of detection of ventricular ectopic beats using ventricular electrogram. This is achieved by detecting the time interval, T, between a point Q defined as the onset of the QRS complex and the peak value of R (T=Q*R) during a heart beat cycle and comparing that time with a running mean value calculated from normal heartbeats.

The object of the present invention is to prevent intrinsic ventricular contraction to occur too early in relation to the atrial contraction. If ventricular contraction occurs "premature" there is not enough time for the necessary atrial blood volume transport resulting in a lowered stroke volume and decreasing heart minute volume.

The above-described problem may occur if the atrial frequency decreases in relation to the ventricular frequency, or if the ventricular frequency increases in relation to the prevailing atrial frequency.

The decrease of the atrial frequency in relation to the ventricular frequency may be caused by:
1) too slow sinus rate during rest (sinus bradycardia).
2) too slow sinus rate acceleration during start of work (chronotropic incompetence).
3) no or too slow sinus rate during work (chronotropic incompetence).
4) too fast decrease of sinus rate at end of work (chronotropic incompetence).
5) Suboptimal atrial stimulation rate increase of a rate responsive pacemaker system, which is sometimes necessary to program in order to avoid unwarranted rate increase due to less specific or improper sensor response, e.g. arm movement without significant workload, etc.

There may be many different reasons to the described problem, e.g. diseases related to the heart and its conduction system or diseases not directly related to the heart, influences from the nervous system, from hormones and/or enzymes (including vasovagal syncope) and/or from medication.

Thus, the general object of the present invention is to achieve an implantable heart stimulator provided with means preventing the PR- and AR-intervals from being too short.

### Summary of the invention

The above-mentioned object is achieved by the present invention according to the independent claim.

Preferred embodiments are set forth in the dependent claims.

The present invention aims to automatically increase the atrial stimulation rate to override possible intrinsic nodal rhythm and thus to restore conditions for appropriate AV-sequential contraction pattern.

In particular, the present invention will provide a benefit for patients suffering from chronotropic incompetence, i.e. a state when the rate increase of the sinus node or the paced atrial rate does not meet the physiological requirement, e.g. during periods of physical exercise. Conditions like that open for compensatory rate increase of the AV-node during physical exercise resulting in less optimal hemodynamics due to compromised atrial transport function and decrease in stroke volume, a problem the present invention aims to solve.
Generally this is solved by the present invention by increasing the atrial stimulation rate until the AR interval has reached a preset value.

According to a preferred embodiment the PR interval is monitored and if the PR interval is shorter than 60 ms the pacemaker begins stimulating the atrium with an atrial stimulating rate that slowly increases until the AR interval is at least 100 ms.

The algorithm may be sensitive to crosstalk. Crosstalk prevails when the ventricular detector senses and interprets noise and disturbances or parts of an atrial stimulation as a QRS. If that is the situation the rate will be falsely increased unless crosstalk prevention measurements are taken.

In particular the present invention is achieved by implementing an AV-nodal rate detection window after each atrial event. Preferably, the lengths of the detection window are 100 ms after an atrial stimulation and 60 ms after a sensed P-wave.
In case ventricular sensing is accomplished during the detection window the occurrence of a premature depolarisation of the ventricles is considered to exist. That will result in an immediate temporary increase of the atrial stimulation rate by e.g. five min⁻¹ for five consecutive cycles. Continued detection of native R-waves during subsequent detection windows will initiate a further increase of the atrial stimulation rate up to a maximum of preferably 100 min⁻¹. The absence of positive detection during five consecutive cycles initiates a stepwise decrease (preferably five min⁻¹) of the temporary increased atrial stimulation rate.

### Short description of the appended drawings

Figure 1 schematically shows a simplified ECG-strip that illustrates a situation where the present invention is applicable.
Figure 2 schematically shows a simplified ECG-strip where the present invention is illustrated.
Figure 3 shows a block diagram of a heart stimulator according to the present invention.

### Detailed description of preferred embodiments of the invention

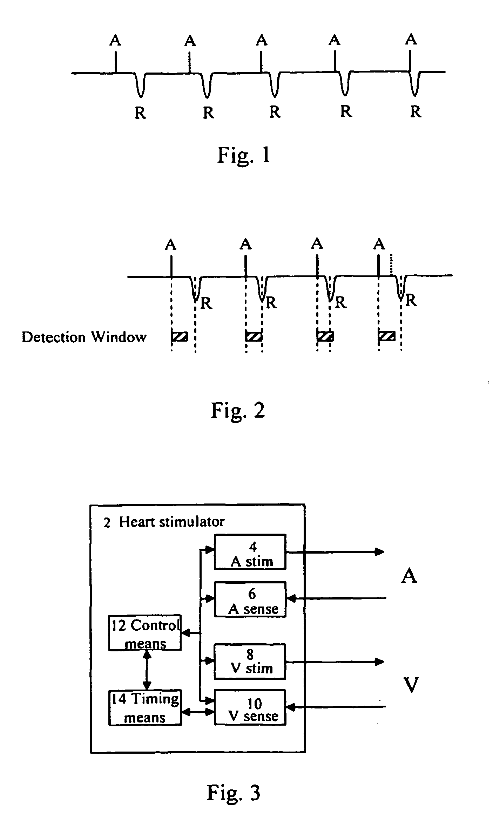

Figure 1 schematically shows a simplified ECG-strip that illustrates a situation where the present invention is applicable.
Figure 1 shows five consecutive heart cycles where atrial stimulation pulses A are applied. These pulses are illustrated by vertical bars. The ventricular depolarization is schematically illustrated by a down-going curve designated by an "R". As is evident from figure 1 the time intervals between A and R gradually decreases between the leftmost events to the rightmost events along the horizontal axis. The rightmost events essentially occur at the same time. As discussed above this may result in less optimal hemodynamic conditions.

With references to figure 3, the present invention relates to a heart stimulator 2 for enhancing cardiac performance of a patient suffering from ventricular rhythms tending to overdrive the atrial rhythm. The stimulator comprises a sensing means 6, 10 for sensing atrial contractions and ventricular contractions, and atrial stimulating means 4 for generating and applying stimulation pulses to the atrium.

The heart stimulator further comprises a timing means 14 for generating a detection window having a predetermined length for said sensing means. The detection window starts upon detection of an atrial depolarisation, intrinsic or stimulated, and if a subsequent ventricular sensing occurs during the ongoing window a "premature" nodal depolarisation of the s the ventricles is considered to exist. This will result in an increase of the atrial stimulation rate for a preset number of consecutive heart cycles.

The present invention provides an additional function to be implemented in dual chamber cardiac stimulation devices aiming for the maintenance of appropriate atrio-ventricular synchrony when "competing" intrinsic nodal activity during an ongoing PV/AV interval occurs.

Preferably, the atrial and ventricular contractions are detected by sensing of the electrical activity of the heart events, the electrogram, related to the depolarization and repolarization of the heart muscle cells.

Alternatively, the atrial and the ventricular contractions may be detected mechanically, e.g. by detecting pressure, movement or sound, or visually, by ultrasound or X-ray techniques.

Figure 3 shows a block diagram of a heart stimulator according to a preferred embodiment of the present invention.
With references to figure 3 the preferred embodiment of the present invention relates to a dual-chamber heart stimulator 2 for enhancing cardiac performance of a patient suffering from ventricular rhythms tending to override the atrial rhythm by shortening the AR interval. The heart stimulator comprises an atrial sensing means 6 for sensing P-waves associated with atrial contractions, ventricular sensing means 10 for sensing R-waves associated with ventricular contractions, atrial stimulating means 4 for generating and applying stimulation pulses to the atrium, and also ventricular stimulating means 8 for generating and applying stimulation pulses to the ventricle.

The heart stimulator further comprises a timing means 14 for generating a detection window having a predetermined length for said ventricular sensing means and a control means 12 responsive for the control of all different parts of the heart stimulator. The heart stimulator also comprises (not shown) battery means for power supply of the heart stimulator, at least two heart electrode leads connectable to the heart stimulator and adapted to be inserted into the heart according to established technique, and communication means for performing e.g. bi-directed communication with an external programming unit.

The detection window is started upon detection of an atrial event, intrinsic or stimulated, and if an R-wave is sensed by the ventricular sensing means during the window a focus that pre-depolarizes the ventricles is considered to exist. In that case the atrial stimulation rate is increased for a preset number of consecutive heart cycles.

This situation is schematically illustrated by the simplified ECG-strip in figure 2. In figure 2, the detection window has lapsed prior the occurrence of the R-wave for the first two heart cycles. In the third heart cycle the R-wave occurs during the detection window and as a consequence the atrial stimulation rate is increased for the next and consecutive heart cycles. This is schematically illustrated by indicating, for the fourth heart cycle, the original position for the stimulation pulse by a dotted vertical line and the applied atrial stimulation pulse at the increased rate by a vertical solid line designated by an "A".

The predetermined detection window length is between 0 and 150 ms, preferably 100 ms, if said atrial event is an atrial stimulation pulse and between 0 and 120 ms, preferably 60 ms, if said atrial event is a sensed P-wave.

According to a preferred embodiment the atrial stimulation rate increase is preferably five stimulation pulses/min during five consecutive heart cycles. The stimulation rate increase may naturally, within the scope of the present invention, be set to a value close to five, e.g. in the interval three to ten stimulation pulses/min in dependence of the prevailing situation.

In a preferred embodiment continued detection of native R-waves during subsequent detection windows initiates a further increase of the atrial stimulation rate up to a maximum rate value, e.g. 100 min -1. This value is variable and set by the physician in relation to the condition of the patient.

The maximum rate value may also be set in dependence of an output signal of an activity sensor (not shown in the figure) such that an increased physical activity of the patient results in an increased maximum rate value.

Thus, according to the present invention if an R-wave is detected in a detection window the atrial stimulation rate is directly increased by e.g. five atrial stimulations per minute during five consecutive heart cycles. If an R-wave still is detected within the detection window and during the five consecutive heart cycles, e.g. in the second of these heart cycles, the rate is then directly further increased.

Absence of native R-wave detections within the detection window during five consecutive heart cycles initiates a stepwise decrease of the increased atrial stimulation rate. Preferably, the stepwise decrease is five stimulation pulses/min in steps of five consecutive heart cycles.

The present invention is not limited to the above-described preferred embodiments. Various alternatives, modifications and equivalents may be used. Therefore, the above embodiments should not be taken as limiting the scope of the invention, which is defined by the appending claims.

## Claims

1. A heart stimulator (2) for enhancing cardiac performance of a patient suffering from ventricular rhythms tending to overdrive the atrial rhythm, comprising sensing means (6, 10) for sensing atrial and ventricular contractions and atrial stimulating means (4) for generating and applying stimulation pulses to the atrium, wherein said heart stimulator further comprises timing means (14) for generating a detection window having a predetermined length for said sensing means, said detection window starts upon detection of an atrial contraction, intrinsic or stimulated, and if a ventricular contraction is sensed by the sensing means during said window a focus that pre-depolarizes the ventricles is considered to exist, wherein the atrial stimulation rate is increased for a preset number of consecutive heart cycles.

2. Heart stimulator according to claim 1, wherein said stimulator is a dual chamber heart stimulator and that said sensing means comprises an atrial sensing means (6) and a ventricular sensing means (10) for sensing electrical activity in the atrium and in the ventricle, respectively.

3. Heart stimulator according to claim 1, wherein the atrial and the ventricular contractions are detected mechanically.

4. Heart stimulator according to claim 1, wherein the atrial and the ventricular contractions are detected mechanically by a pressure sensor.

5. Heart stimulator according to claim 1, wherein said predetermined detection window length is between 0 and 150 ms if said atrial contraction is caused by an atrial stimulation pulse and between 0 and 120 ms if said atrial contraction is an intrinsic contraction.

6. Heart stimulator according to claim 1, wherein said predetermined detection window length is 100 ms if said atrial event is an atrial stimulation pulse and 60 ms if said atrial event is a sensed P-wave.

7. Heart stimulator according to claim 1, wherein said atrial stimulation rate increase is five stimulation pulses/min during five consecutive heart cycles.

8. Heart stimulator according to claim 1, wherein continued detection of native R-waves during subsequent detection windows initiates a further increase of the atrial stimulation rate up to a maximum rate value.

9. Heart stimulator according to claim 5, wherein said maximum rate value is 100 min -1.

10. Heart stimulator according to claim 9, wherein said maximum rate value is set in dependence of an output signal of an activity sensor such that an increased physical activity of the patient results in an increased maximum rate value.

11. Heart stimulator according to claim 1, wherein the absence of native R-wave detections during five consecutive heart cycles initiates a stepwise decrease of the increased atrial stimulation rate.

12. Heart stimulator according to claim 11, wherein said decrease is five stimulation pulses/min in steps of five consecutive heart cycles.

## Patentansprüche

1. Herzstimulator (2) zum Verbessern der Herzleistung eines Patienten, der daran leidet, dass Herzkammerrhythmen zu einem Overdrive für den Vorhofrhythmus neigen, wobei der Herzstimulator ein Abtastmittel (6, 10) zum Erkennen von Vorhof- und Herzkammerkontraktionen und ein Vorhofstimulationsmittel (4) zum Erzeugen und Anwenden von Stimulationsimpulsen auf den Vorhof umfasst, wobei der Herzstimulator ferner ein Zeitgebermittel (14) zum Erzeugen eines Erfassungsfensters einer vorgegebenen Länge für das Abtastmittel umfasst, wobei das Erfassungsfenster nach dem Erfassen einer stimulierten oder unstimulierten Vorhofkontraktion beginnt, und, wenn von dem Abtastmittel während des Zeitfensters eine Herzkammerkontraktion erkannt wird, angenommen wird, dass ein Fokus existiert, der die Herzkammern vordepolarisiert, wobei die Vorhofstimulationsrate für eine vorab eingestellte Anzahl aufeinander folgender Herzzyklen erhöht wird.

2. Herzstimulator nach Anspruch 1, wobei es sich bei dem Stimulator um einen Zweikammer-Herzstimulator handelt und wobei das Abtastmittel ein Vorhof-Abtastmittel (6) und ein Herzkammer-Abtastmittel (10) zum Abtasten der elektrischen Aktivität in dem Vorhof bzw. in der Herzkammer umfasst.

3. Herzstimulator nach Anspruch 1, wobei die Vorhof- und Herzkammerkontraktionen mechanisch erfasst werden.

4. Herzstimulator nach Anspruch 1, wobei die Vorhof- und Herzkammerkontraktionen mechanisch durch einen Drucksensor erfasst werden.

5. Herzstimulator nach Anspruch 1, wobei die vorgegebene Länge des Erfassungsfensters 0 ms bis 150 ms beträgt, wenn die Vorhofkontraktion von einem Vorhofstimulationsimpuls verursacht wird, und 0 ms bis 120 ms beträgt, wenn es sich bei der Vorhofkontraktion um eine unstimulierte Kontraktion handelt.

6. Herzstimulator nach Anspruch 1, wobei die vorgegebene Länge des Erfassungsfensters 100 ms beträgt, wenn es sich bei dem Vorhofereignis um einen Vorhofstimulationsimpuls handelt, und 60 ms beträgt, wenn es sich bei dem Vorhofereignis um eine erkannte P-Zacke handelt.

7. Herzstimulator nach Anspruch 1, wobei die Erhöhung der Vorhofstimulationsrate fünf Stimulationsimpulse je Minute während fünf aufeinander folgender Herzzyklen beträgt.

8. Herzstimulator nach Anspruch 1, wobei die anhaltende Erfassung von natürlichen R-Zacken während aufeinander folgender Erfassungsfenster eine weitere Erhöhung der Vorhofstimulationsrate bis zu einem Maximalratenwert auslöst.

9. Herzstimulator nach Anspruch 5, wobei der Maximalratenwert 100 min⁻¹ beträgt.

10. Herzstimulator nach Anspruch 9, wobei der Maximalratenwert in Abhängigkeit von einem Ausgangssignal aus einem Aktivitätssensor eingestellt wird, derart, dass eine erhöhte körperliche Aktivität des Patienten zu einem erhöhten Maximalratenwert führt.

11. Herzstimulator nach Anspruch 1, wobei die fehlende Erfassung von natürlichen R-Zacken während fünf aufeinander folgender Herzzyklen eine schrittweise Verringerung der erhöhten Vorhofstimulationsrate auslöst.

12. Herzstimulator nach Anspruch 11, wobei die Verringerung fünf Stimulationsimpulse je Minute in Schritten von fünf aufeinander folgenden Herzzyklen beträgt.

## Revendications

1. Stimulateur cardiaque (2) pour augmenter les performances cardiaques d'un patient présentant des rythmes ventriculaires qui ont tendance à augmenter la fréquence du rythme auriculaire, comprenant des moyens de détection (6, 10) pour détecter les contractions auriculaires et ventriculaires et des moyens de stimulation auriculaire (4) pour générer et appliquer des impulsions de stimulation à l'oreillette, ledit stimulateur cardiaque comprenant en outre des moyens de synchronisation (14) pour générer une fenêtre de détection ayant une longueur prédéterminée pour lesdits moyens de détection, laquelle fenêtre de détection commence au moment de la détection d'une contraction auriculaire intrinsèque ou stimulée, et, si une contraction ventriculaire est détectée par les moyens de détection dans ladite fenêtre, on considère qu'il existe un foyer qui pré-dépolarise les ventricules, la fréquence de stimulation auriculaire étant augmentée pendant un nombre prédéfini de cycles cardiaques consécutifs.

2. Stimulateur cardiaque selon la revendication 1, dans lequel ledit stimulateur est un stimulateur cardiaque à double chambre et lesdits moyens de détection comprennent des moyens de détection auriculaire (6) et des moyens de détection ventriculaire (10) pour détecter l'activité électrique de l'oreillette et du ventricule, respectivement.

3. Stimulateur cardiaque selon la revendication 1, dans lequel les contractions auriculaires et ventriculaires sont détectées mécaniquement.

4. Stimulateur cardiaque selon la revendication 1, dans lequel les contractions auriculaires et ventriculaires sont détectées mécaniquement par un capteur de pression.

5. Stimulateur cardiaque selon la revendication 1, dans lequel ladite longueur prédéterminée de la fenêtre de détection va de 0 à 150 ms si ladite contraction auriculaire est provoquée par une impulsion de stimulation auriculaire et de 0 à 120 ms si ladite contraction auriculaire est une contraction intrinsèque.

6. Stimulateur cardiaque selon la revendication 1, dans lequel ladite longueur prédéterminée de la fenêtre de détection est de 100 ms si ledit événement auriculaire est une impulsion de stimulation auriculaire et de 60 ms si ledit événement auriculaire est une onde P détectée.

7. Stimulateur cardiaque selon la revendication 1, dans lequel ladite augmentation de la fréquence de stimulation auriculaire est de cinq impulsions de stimulation par minute pendant cinq cycles cardiaques consécutifs.

8. Stimulateur cardiaque selon la revendication 1, dans lequel la détection continue d'ondes R natives dans des fenêtres de détection suivantes déclenche une augmentation supplémentaire de la fréquence de stimulation auriculaire jusqu'à une valeur de fréquence maximale.

9. Stimulateur cardiaque selon la revendication 5, dans lequel ladite valeur de fréquence maximale est égale à 100 min⁻¹.

10. Stimulateur cardiaque selon la revendication 9, dans lequel ladite valeur de fréquence maximale est fixée en fonction d'un signal de sortie d'un capteur d'activité de telle façon qu'une augmentation de l'activité physique du patient entraîne une augmentation de la valeur de fréquence maximale.

11. Stimulateur cardiaque selon la revendication 1, dans lequel l'absence de détection d'ondes R natives pendant cinq cycles cardiaques consécutifs déclenche une diminution par paliers de la fréquence de stimulation auriculaire augmentée.

12. Stimulateur cardiaque selon la revendication 11, dans lequel ladite diminution est de cinq impulsions de stimulation par minute par paliers de cinq cycles cardiaques consécutifs.
